# EUROPÄISCHE PATENTSCHRIFT

(11) **EP 2 632 443 B1**
(45) Veröffentlichungstag und Bekanntmachung des Hinweises auf die Patenterteilung: **17.09.2014**
(21) Anmeldenummer: 11782080.3
(22) Anmeldetag: 27.10.2011
(51) Int. Cl.: A61K 9/70

(54) **HERSTELLUNG ORODISPERSIBLER FILME**
PREPARATION OF ORODISPERSIBLE FILMS
PRÉPARATION DE FILMS ORODISPERSIBLES

(30) Priorität: 28.10.2010 DE 102010049706
(43) Veröffentlichungstag der Anmeldung: 04.09.2013
(73) Patentinhaber: Hexal AG, 83607 Holzkirchen (DE)
(72) Erfinder: KREKELER, Andreas, 83607 Holzkirchen (DE); NEUMANN, Dimitri, 83607 Holzkirchen (DE)
(74) Vertreter: Baumann, Rüdiger Walter
(86) Internationale Anmeldenummer: PCT/EP2011/068825
(87) Internationale Veröffentlichungsnummer: WO 2012/055947

(56) Entgegenhaltungen:
- EP-A2- 0 614 659
- WO-A1-2008/108940
- WO-A2-00/42992
- WO-A2-2007/009801
- US-A1- 2004 043 061

## Beschreibung

### Gebiet der Erfindung

Die vorliegende Erfindung betrifft ein Verfahren zur Herstellung einer pharmazeutischen Zusammensetzung, die insbesondere für die Bildung eines orodispersiblen Films geeignet ist sowie eine Zusammensetzung, insbesondere in Form eines orodispersiblen Films.

### Hintergrund der Erfindung

Die perorale Gabe von Arzneistoffen stellt nach wie vor die am häufigsten angewandte Art der Verabreichung von Medikamenten dar. Für die Compliance der Patienten bei der peroralen Gabe sind der Geschmacks- und der Geruchssinn des Menschen von großer Bedeutung.

Traditionelle Darreichungsformen sind beispielsweise Tabletten oder Kapseln, die als Trägersysteme für die orale Verabreichung von Arzneistoffen verwendet werden. Die Tabletten oder Kapseln werden dabei in der Regel geschluckt, was bedingt, dass der Patient eine Flüssigkeit bereit hält, mit der er diese Darreichungsform einnehmen kann.

Teilweise bestehen jedoch gerade bei älteren Patienten und Kindern Schluckbeschwerden, sodass diese die Einnahme von Tabletten oder Kapseln ablehnen bzw. eine Einnahme nur ungern erfolgt. Hieraus resultiert dann nicht selten eine schlechte Compliance, die für den Heilungsfortschritt bzw. den Therapieerfolg nachteilige Auswirkungen hat.

Auch bei Patientengruppen mit psychischen Erkrankungen, bei denen die Überwachung der tatsächlichen Einnahme ihrer Medikation unabdingbar ist, ist die Verabreichung von herkömmlichen Darreichungsformen wie Tabletten oder Kapseln nicht unproblematisch. Aufgrund der verzögerten Auflösung können derartige Wirkstoffträger leicht aus dem Mund entfernt werden, ohne dass es dem überwachenden medizinischen Personal auffällt.

Um die beschriebenen Probleme zu überwinden, sind pharmazeutische Darreichungsformen, wie z. B. Granulate oder orale Filme entwickelt worden, die ohne Flüssigkeitszufuhr eingenommen werden können und die in der Mundhöhle rasch zerfallen. Orale Filme zeichnen sich beispielsweise dadurch aus, dass sie eine geringe Schichtdicke und eine große Oberfläche aufweisen und in kürzester Zeit in der Mundhöhle zerfallen. Sie können jederzeit und überall je nach Bedarf des Patienten auch diskret eingenommen werden. Eine gleichzeitige Einnahme von Flüssigkeit ist nicht notwendig, da die in der Mundhöhle vorhandene Speichelflüssigkeit ausreicht, um die Filme aufzulösen und den Wirkstoff freizusetzen.

Orale Filme, die pharmazeutische und nichtpharmazeutische Wirkstoffe enthalten sowie Verfahren zu deren Herstellung werden z. B. beschrieben in der WO 2007/009800, WO 2007/009801 und WO 03/011259.

Ein besonderes Problem bei der Verabreichung herkömmlicher, wenigstens einen pharmazeutischen Inhalts- oder Wirkstoff enthaltender oraler Filme ist zum Einen der oft unangenehme und zuweilen als extrem bitter empfundene Geschmack der darin eingebrachten Inhaltsstoffe, zum Anderen die Tatsache, dass die in herkömmlichen Verfahren hergestellten Filme bezüglich des absoluten Wirkstoffgehalts nur eine begrenzte Kapazität aufweisen. Dies liegt daran, dass bei herkömmlicherweise hergestellten oralen Filmen der Wirkstoff, sofern dieser hochdosiert im Medikament enthalten sein soll, zunächst in einem Lösungsmittelsystem, in dem der Wirkstoff gut löslich ist, gelöst wird. Orale Filme, die beispielsweise einen hochdosierten, sehr gut wasserlöslichen Wirkstoff enthalten, werden gemäß den aus dem Stand der Technik bekannten Verfahren in einem wässrigen Lösungssystem gelöst. Gerade hierbei zeigt es sich jedoch als nachteilig, dass sich orale Filme, die einen, in einem wässrigen Lösungsmittel gelösten, gut wasserlöslichen Wirkstoff enthalten, nicht zu homogenen Laminaten trocknen lassen. Dieser Nachteil zeigt sich insbesondere dann, wenn der Wirkstoff in hoher Konzentration homogen verteilt im Film vorliegen soll. Der Wirkstoff löst sich durch den Wärmeeintrag bei der Trocknung in der Restmatrix teilweise bzw. vollständig auf und/oder bildet eine konzentrierte Salzlösung, die sich nicht mehr trocknen lässt. Es kann auch zur Bildung von Wirkstoffinseln im Film kommen, sodass eine homogene Verteilung der Inhaltsstoffe im den Film bildenden Laminat nicht gewährleistet ist.

Aufgabe der vorliegenden Erfindung war es, ein Verfahren zur Herstellung einer pharmazeutischen Zusammensetzung bereit zu stellen, wobei die Zusammensetzung insbesondere zur oralen Applikation geeignet ist, hohe Wirkstoffkonzentrationen aufweist und der Wirkstoff homogen in der Zusammensetzung verteilt ist. Aufgabe war es weiterhin, eine Zusammensetzung insbesondere in Form eines orodispersiblen Films zur Verfügung zu stellen.

### Zusammenfassung der Erfindung

Die vorliegende Erfindung betrifft folgende Gegenstände:
1. Verfahren zur Herstellung einer Zusammensetzung, insbesondere einer pharmazeutischen oder kosmetischen Zusammensetzung, bevorzugt einer pharmazeutischen oder kosmetischen Zusammensetzung zur oralen Applikation, bevorzugt in Form eines orodispersiblen Films, umfassend die Schritte:
   (i) Bilden einer Suspension aus wenigstens einem pharmazeutischen Inhaltsstoff (A) und einem Lösungsmittel oder Lösungsmittelgemisch (B), wobei der wenigstens eine pharmazeutische Inhaltsstoff (A) in dem Lösungsmittel oder Lösungsmittelgemisch (B) schwer löslich oder nicht löslich ist,
   (ii) Zugabe wenigstens eines Gelbildners (C) zu der Suspension, wobei der wenigstens eine Gelbildner (C) in dem Lösungsmittel oder Lösungsmittelgemisch (B) quellbar ist, und optional
   (iii) Quellen der Suspension.
2. Verfahren nach Gegenstand 1, ferner umfassend die Schritte:
   (iv) Bilden eines Films aus der aus Schritt (ii) oder Schritt (iii) erhältlichen Suspension und
   (v) Trocknen des Films.
3. Verfahren nach Gegenstand 1 oder 2, weiterhin umfassend die Zugabe wenigstens einer weiteren Substanz ausgewählt aus der Gruppe bestehend aus Aroma, Süßstoff und Weichmacher, wobei die wenigstens eine weitere Substanz in dem Lösungsmittel oder Lösungsmittelgemisch (B) leicht löslich ist.
4. Verfahren nach einem der vorhergehenden Gegenstände,
   dadurch gekennzeichnet, dass
   der pharmazeutische Inhaltsstoff (A) ein pharmazeutisch wirksamer Inhaltsstoff oder eine Mischung umfassend wenigstens einen pharmazeutisch wirksamen Inhaltsstoff ist.
5. Verfahren nach einem der vorhergehenden Gegenstände,
   dadurch gekennzeichnet, dass
   - ein Anteil des wenigstens einen pharmazeutischen Inhaltsstoffes (A) zwischen 10 Gew.% und 80 Gew.%, bevorzugt zwischen 50 Gew.% und 70 Gew.%,
   - ein Anteil des Gelbildners (C) zwischen 5 Gew.% und 25 Gew.%, bevorzugt bei 7,5 Gew.%,
      jeweils bezogen auf das Trockengewicht der Zusammensetzung, liegt.
6. Verfahren nach Gegenstand 3,
   dadurch gekennzeichnet, dass
   - ein Anteil des Weichmachers zwischen 2 Gew.% und 15 Gew.%, bevorzugt bei 7,5 Gew.%,
   - ein Anteil des Aromastoffes zwischen 0 Gew.% und 10 Gew.%, bevorzugt bei 7 Gew.%, und/oder
   - ein Anteil des Süßungsmittels zwischen 0 Gew.% und 5 Gew.%, bevorzugt bei 4 Gew.%,
      jeweils bezogen auf das Trockengewicht der Zusammensetzung, liegt.
7. Verfahren nach einem der vorhergehenden Gegenstände,
   dadurch gekennzeichnet, dass
   das Lösungsmittelgemisch (B) wenigstens zwei Lösungsmittel umfasst, wobei der Anteil der Lösungsmittel im Gemisch zwischen 50% (v/v) bis 100% (v/v) beziehungsweise 0% (v/v) bis 50% (v/v) beträgt.
8. Verfahren nach einem der vorhergehenden Gegenstände,
   dadurch gekennzeichnet, dass
   der pharmazeutische Inhaltsstoff (A) in einem wässrigen Lösungsmittelsystem oder wässrigen Lösungsmittelsystemen oder Mischungen daraus leicht löslich ist und das Lösungsmittel oder Lösungsmittelgemisch (B) ausgewählt ist aus C1-C5 Alkanolen, insbesondere Ethanol oder Isopropanol, Aceton oder Mischungen daraus.
9. Verfahren nach einem der vorhergehenden den Gegenstände,
   dadurch gekennzeichnet, dass
   der pharmazeutische Inhaltsstoff (A) in einem wässrigen Lösungsmittelsystem oder wässrigen Lösungsmittelsystemen oder Mischungen daraus leicht löslich ist und das Lösungsmittel (B) ein Lösungsmittelgemisch umfassend Aceton und Ethanol, insbesondere ein Lösungsmittelgemisch umfassend Aceton und Ethanol mit einem Anteil an Aceton zwischen 50% (v/v) und 95% (v/v) und einem Anteil an Ethanol zwischen 5% (v/v) und 50% (v/v), bezogen auf das Lösungsmittelgemisch, ist.
10. Verfahren nach einem der vorhergehenden den Gegenstände,
   dadurch gekennzeichnet, dass
   der Gelbildner (C) ausgewählt ist aus der Gruppe bestehend aus Hydroxypropylcellulose, Hydroxypropylmethylcellulose, Methylcellulose, Klucel®, Klucel® E, Klucel® L, Klucel® J, Klucel® G, Klucel® M, Klucel® H, Klucel® EF, Klucel® LF, Klucel® JF, Klucel® GF, Klucel® MF, Klucel® HF, Klucel® EXF, Klucel® LXF, Klucel® JXF, Klucel® GXF, Klucel® MXF, Klucel® HXF, Ethylcellulose oder Mischungen daraus.
11. Verfahren nach einem der vorhergehenden den Gegenstände,
   dadurch gekennzeichnet, dass
   der pharmazeutische Inhaltsstoff (A) ausgewählt ist aus der Gruppe bestehend aus Sumatriptan, Sildenafil, Acetylcystein, Ambroxol, Citalopram, Clopidogrel, Losartan, Mitrazapin, Valproinsäure, Verapamil, pharmazeutisch akzeptablen Salzen davon oder Mischungen daraus, und insbesondere ausgewählt ist aus Sumatriptan-Succinat und Sildenafil-Citrat.
12. Verfahren nach Gegenstand 11,
   dadurch gekennzeichnet, dass
   der Gehalt an Sumatriptan-Succinat bzw. Sildenafil-Citrat bei 30-70 Gew.%, bevorzugt 50-65 Gew.%, bezogen auf das Trockengewicht der Zusammensetzung, liegt.
13. Verfahren nach einem der vorhergehenden Gegenstände 3 bis 12,
   dadurch gekennzeichnet, dass
   der Weichmacher ausgewählt ist der Gruppe bestehend aus Glycerol, Propylenglycol, Dibutylsebacat, Triacetin, Triethylcitrat und Isopropylmyristat sowie Mischungen daraus, bevorzugt aus Glycerol und Propylenglycol.
14. Verfahren nach einem der vorhergehenden Gegenstände 2 bis 13,
   dadurch gekennzeichnet, dass die Bildung des Films in einem Gieß-, Zieh-, Extrusions- oder Sprühverfahren und die Trocknung des Films durch Wärmebeaufschlagung erfolgt.
15. Verfahren nach einem der vorhergehenden Gegenstände 2 bis 14,
   dadurch gekennzeichnet, dass
   der Film eine Nassschichtdicke von 400 µm bis 600 µm, bevorzugt von 460 µm bis 490 µm und/oder eine Trockenschichtdicke von 100 µm bis 200 µm, bevorzugt von 140 µm bis 180 µm aufweist.
16. Verfahren zur Herstellung einer pharmazeutischen Zusammensetzung, insbesondere einer Zusammensetzung zur oralen Applikation, bevorzugt in Form eines orodispersiblen Films, umfassend die Schritte:
   (i) Bereitstellen eines Lösungsmittels oder eines Lösungsmittelgemisches (B),
   (ii) Zugabe wenigstens eines Süßstoffes, wenigstens eines Weichmachers und optional wenigstens eines Aromastoffes zu dem Lösungsmittel oder Lösungsmittelgemisch (B),
   (iii) Suspendieren wenigstens eines pharmazeutischen Inhaltsstoffes (A) in dem aus Schritt (ii) erhältlichen Gemisch,
   (iv) Zugabe wenigstens eines in dem Lösungsmittel oder Lösungsmittelgemisch (B) quellbaren Gelbildners (C) zu dem aus Schritt (iii) erhältlichen Gemisch,
   (v) Quellen der aus Schritt (iv) erhältlichen Suspension,
   (vi) Bilden eines Filmes aus der aus Schritt (v) erhältlichen Suspension,
   (vii) Trocknen des Filmes,
      wobei der wenigstens eine pharmazeutische Inhaltsstoff (A) in dem Lösungsmittel oder Lösungsmittelgemisch (B) schwer löslich oder nicht löslich ist, und wobei das Lösungsmittel oder Lösungsmittelgemisch (B) bevorzugt wie in einem der Gegenstände 7 bis 9 definiert ist.
17. Pharmazeutische Zusammensetzung, insbesondere pharmazeutische Zusammensetzung in Form eines orodispersiblen Films, hergestellt in einem Verfahren gemäß einem der Gegenstände 1 bis 16.
18. Pharmazeutische Zusammensetzung nach Gegenstand 17, gekennzeichnet durch
   einen Restgehalt des Lösungsmittels bzw. der Lösungsmittel (B) von weniger als 5000ppm, bevorzugt weniger als 2000ppm bezogen auf das Trockengewicht des Films.
19. Pharmazeutische Zusammensetzung, nach Gegenstand 17 oder 18,
   dadurch gekennzeichnet, dass
   die pharmazeutische Zusammensetzung in Form eines oralen Films vorliegt und der orale Film wasserfrei oder im wesentlichen wasserfrei ist und der wenigstens eine pharmazeutische Inhaltsstoff (A) in Wasser oder in einem wässrigen Lösungsmittelgemisch, einschließlich Speichel, leicht löslich ist und in dem für die Herstellung des Films verwendeten Lösungsmittel oder Lösungsmittelgemisch (B), ausgewählt aus C1-C5 Alkanolen, insbesondere Ethanol oder Isopropanol, Aceton oder Mischungen daraus, schwer löslich oder nicht löslich ist.
20. Pharmazeutische Zusammensetzung umfassend wenigstens einen pharmazeutisch aktiven Inhaltsstoff ausgewählt aus der Gruppe bestehend aus Sumatriptan, Sildenafil, Acetylcystein, Ambroxol, Citalopram, Clopidogrel, Losartan, Mitrazapin, Valproinsäure, Verapamil, pharmazeutisch akzeptablen Salzen davon oder Mischungen daraus, und insbesondere ausgewählt aus Sumatriptan-Succinat und Sildenafil-Citrat, gekennzeichnet durch einen Wasseranteil der pharmazeutischen Zusammensetzung von weniger als 0,5 Gew.% und einen Anteil an Lösungsmittel ausgewählt aus C1-C5 Alkanolen, insbesondere Ethanol oder Isopropanol, Aceton oder Mischungen daraus von 2000ppm oder weniger, wobei der Anteil des wenigstens einen pharmazeutisch aktiven Inhaltsstoffes zwischen 50 Gew.% und 70 Gew.% liegt.

### Ausführliche Beschreibung der Erfindung

Die vorliegende Erfindung betrifft ein Verfahren zur Herstellung einer Zusammensetzung, insbesondere einer Zusammensetzung zur oralen Applikation, wobei die Zusammensetzung einen pharmazeutischen bzw. pharmazeutisch wirksamen Inhaltsstoff umfasst. Das erfindungsgemäße Verfahren umfasst die Schritte:
(i) Bildung einer Suspension aus wenigstens einem Inhaltsstoff (A) und einem Lösungsmittel oder Lösungsmittelgemisch (B), wobei der wenigstens eine Inhaltsstoff (A) in dem Lösungsmittel oder Lösungsmittelgemisch (B) schwer löslich oder nicht löslich ist,
(ii) Zugabe wenigstens eines Gelbildners (C) zu der aus Schritt (i) erhältlichen Suspension, wobei der wenigstens eine Gelbildner dahingehend ausgewählt ist, dass dieser in dem Lösungsmittel oder Lösungsmittelgemisch (B) quellbar ist.
   In einer bevorzugten Ausführungsform umfasst das Verfahren des Weiteren ein
(iii) Quellen der aus Schritt (ii) resultierenden Suspension.

Hierbei wird die Quelldauer anhand des verwendeten Gelbildners sowie des bestimmungsgemäßen Verwendungszweckes der Suspension bzw. des gebildeten Gels bestimmt. Über die Art des Gelbildners sowie die Quelldauer und die Behandlung der Suspension während des Quellvorganges, beispielsweise durch Rühren oder Schütteln, können die Eigenschaften des gebildeten Gels bzw. eines daraus gebildeten Films oder dergleichen beeinflusst werden. Die Zusammensetzung kann bereits in der Darreichungsform einer Suspension, als angedickte, bevorzugt gelartige Suspension, als Gel oder als suspensions- oder gelgefüllte Kapsel, beispielsweise in Form einer Hart- oder Weichgelatinekapsel, verabreicht werden.

In einer bevorzugten Weiterbildung des Verfahrens wird aus der Suspension ein Film gebildet. Das Verfahren umfasst dann ferner die Schritte:
(iv) Bilden eines Filmes aus der aus Schritt (ii) oder (iii) erhältlichen Suspension, und
(v) Trocknen des Films.

Hiernach kann der solchermaßen gebildete Film in die letztendliche Film- bzw. Filmabschnittform, beispielsweise in eine runde, beliebig gerundete, ovale, ellipsenförmige, dreieckige, viereckige bzw. quadratische oder rechteckige oder vieleckige Form gebracht werden.

Bei dem im erfindungsgemäßen Verfahren eingesetzten und zu formulierenden pharmazeutischen Inhaltsstoff handelt es sich bevorzugt um einen pharmazeutisch wirksamen Inhaltsstoff (hierin auch als Wirkstoff bezeichnet) oder eine Mischung, die wenigstens einen pharmazeutisch wirksamen Inhaltsstoff umfasst. Selbstverständlich besteht hier auch die Möglichkeit, dass zwei und mehr pharmazeutische Inhaltsstoffe und/oder pharmazeutisch wirksame Inhaltsstoffe gegebenenfalls in unterschiedlichen Mengenanteilen in der Mischung vorliegen. Es kann neben wenigstens einem pharmazeutischen oder pharmazeutisch wirksamen Inhaltsstoff jedoch auch wenigstens ein weiterer pharmazeutisch nicht wirksamer Inhaltsstoff oder sonstiger Inhaltsstoff vorgesehen werden.

Als zu formulierender pharmazeutischer Inhaltsstoff werden im Zusammenhang mit der vorliegenden Erfindung sämtliche Stoffe und Substanzen bezeichnet, die in einer pharmazeutischen und/oder einer der pharmazeutischen Verwendung nahestehenden Verwendung, beispielsweise eine kosmetische Verwendung oder zu diätetischen Zwecken, eingesetzt werden, und die in entsprechenden Zusammensetzungen enthalten sein können. Hierzu zählen beispielsweise auch Konservierungsstoffe, Farbstoffe, Vitamine, Spurenelemente, Nährelemente, Mikronährelemente und Feuchthaltemittel.

Als pharmazeutisch wirksamer Inhaltsstoff werden im Zusammenhang mit der vorliegenden Erfindung Substanzen bezeichnet, die in geringer Dosis in einem Organismus eine spezifische Wirkung oder eine Reaktion hervorrufen. Als pharmazeutisch wirksamer Inhaltsstoff im Zusammenhang mit der vorliegenden Erfindung sind Stoffe zu verstehen, denen eine arzneiliche Wirkung zugeschrieben wird. Neben natürlich vorkommenden Wirkstoffen werden hierunter auch partialsynthetische Abkömmlinge und totalsynthetisch bzw. biotechnologisch hergestellte chemische Wirkstoffe verstanden. Daneben fallen auch Adjuvantien oder Wirkverstärker im Zusammenhang mit der vorliegenden Erfindung unter diese Definition.

Vor dem Verfahrensschritt (iv) Bildung des Filmes bzw. nach dem Verfahrenschritt (ii) Zugabe des Gelbildners kann gegebenenfalls ein (iii) Quellen der Suspension durchgeführt werden. Die Suspension wird dabei bevorzugt mindestens 12 Stunden quellen gelassen, sodass der der Suspension beigegebene Gelbildner günstigerweise vollständig ausquellen kann. Die Quelldauer richtet sich hierbei nach dem jeweils verwendeten Gelbildner bzw. dem gewünschten Quellgrad. Letzterer ist abhängig von der angestrebten Verwendung der im erfindungsgemäßen Verfahren hergestellten Zusammensetzung. Ist die Verwendung in Form eines Gels, einer Paste oder dergleichen vorgesehen, kann eine kürzere Quelldauer oder ein geringerer Gehalt des Gelbildners gewählt werden, um die Konsistenz der Zusammensetzung zu definieren. Die Auswahl beeinflusst somit die Länge des Quellvorganges. Das Quellen kann hierbei unter Rühren oder bei ruhender Suspension erfolgen. Hieraus ergeben sich dann auch Vorteile für die Filmbildung, d. h. für die Ausbildung eines homogenen, orodispersiblen Films mit gleichmäßiger Struktur und Textur sowie gleichmäßiger Verteilung der Inhaltsstoffe, pharmazeutischen Inhaltsstoffe und/oder pharmazeutisch wirksamen Inhaltsstoffe oder Mischungen aus diesen.

Der pharmazeutische bzw. pharmazeutisch wirksame Inhaltsstoff ist in einer Ausführungsform dadurch gekennzeichnet, dass er in einem wässrigen Lösungssystem oder wässrigen Lösungssystemen oder Mischungen daraus leicht löslich ist. Beispielsweise ist der Inhaltsstoff leicht löslich in Wasser. Daneben wird auch die im Mundraum eines Patienten vorliegende Flüssigkeit von den hier genannten wässrigen Lösungsmitteln oder Lösungsmittelgemischen umfasst.

Ist der zu formulierende Inhaltsstoff ein gut wasserlöslicher Inhaltsstoff, so werden als Lösungsmittel oder Lösungsmittelgemisch (B) im erfindungsgemäßen Verfahren bevorzugt Lösungsmittel bzw. Lösungsmittelgemische verwendet, die ausgewählt sind aus C1- bis C5-Alkanolen und hierbei insbesondere organische Lösungsmittel, beispielsweise Ethanol oder Isopropanol oder Aceton. Selbstverständlich können auch Mischungen der vorgenannten C1- bis C5-Alkanole, insbesondere von Ethanol oder Isopropanol und/oder Mischungen mit Aceton oder reines Aceton Verwendung finden.

In einer weiteren Ausführungsform der Erfindung ist der zu formulierende Inhaltsstoffen einem wässrigen Lösungsmittel(system) nicht löslich oder schwer löslich. In diesem Fall ist das Lösungsmittel bzw. Lösungsmittelgemisch (B) ein wässriges Lösungsmittel bzw. Lösungsmittelgemisch, bevorzugt Wasser.

In jedem Fall wird ein Lösungsmittel bzw. Lösungsmittelgemisch verwendet, in welchem der zu formulierende Inhaltsstoff bzw. Wirkstoff nicht oder nur schwer löslich ist. Es kann so erreicht werden, dass sich der Inhaltsstoff/Wirkstoff während der Herstellung der Zusammensetzung und hierbei insbesondere bei der Bildung eines orodispersiblen Films nicht im Lösungsmittel auflöst, sondern stattdessen dispergiert vorliegt. Dadurch wird verhindert, dass der zu formulierende Inhaltsstoff bei einer etwaigen Trocknung der Zusammensetzung eine konzentrierte Salzlösung bildet, die sich nicht mehr trocknen lässt. Zudem wird verhindert, dass sich bei einer Trocknung der Zusammensetzung ungleichmäßige Wirkstoffinseln in der Zusammensetzung bilden, sodass keine homogene Verteilung des Wirkstoffes innerhalb der Zusammensetzung, beispielsweise über den gesamten orodispersiblen Film, gegeben ist.

Das erfindungsgemäße Verfahren kann weiterhin die Zugabe wenigstens einer weiteren Substanz umfassen. Diese weitere Substanz ist bevorzugt ausgewählt aus der Gruppe bestehend aus Aromastoffen, Süßstoffen und Weichmachern, wobei die wenigstens eine weitere Substanz in dem Lösungsmittel oder Lösungsmittelgemisch (B) bevorzugt leicht löslich ist.

Die Begriffe "leicht löslich" und "schwer löslich" bzw. "nicht löslich" wie hierin verwendet, leiten sich bevorzugt von einer Löslichkeitsklassifizierung ab, wobei der Bereich leicht löslicher Stoffe begrenzt ist auf eine Löslichkeit von 0,1 bis > 1 mol/l, und als schwer löslich bzw. nicht löslich eine Löslichkeit von < 0,1 mol/l angesehen wird. Als günstig wird in diesem Zusammenhang angesehen, wenn der wenigstens eine pharmazeutische Inhaltsstoff, dessen Verwendung im erfindungsgemäßen Verfahren vorgesehen ist, in dem Lösungsmittel oder Lösungsmittelgemisch (B) eine Löslichkeit von < 0,1 mol/l aufweist.

Es wird als besonders günstig angesehen, wenn die weitere(n) Substanz(en) vor, nach oder gleichzeitig mit dem pharmazeutischen bzw. pharmazeutisch wirksamen Inhaltsstoff dem Lösungsmittel oder Lösungsmittelgemisch (B) beigegeben wird/werden. Aroma, Süßstoff und/oder Weichmacher können beispielsweise vor der Zugabe des Inhaltsstoffes oder Gelbildners im Lösungsmittel oder Lösungsmittelgemisch (B) bzw. in einem Gemischpartner des Lösungsmittelgemisches (B) gelöst und/oder dispergiert werden. Das so erhältliche Gemisch kann dann, gegebenenfalls nach Zugabe eines weiteren oder mehrerer weiterer Gemischpartner(s) des Lösungsmittelgemisches (B), als Lösungsmittel für die Suspension, verwendet werden. Selbstverständlich können weitere Substanzen auch später, d.h. beispielsweise. nach der Zugabe von pharmazeutischem Inhaltsstoff und/oder Gelbildner, der Suspension beigefügt werden.

In einer vorteilhaften Weiterbildung des erfindungsgemäßen Verfahrens ist vorgesehen, dass die resultierende Zusammensetzung als orodispersibler Film ausgebildet ist und hierin der wenigstens eine Inhaltsstoff in einem Gewichtsanteil von 10 Gew.% bis 80 Gew.%, bevorzugt von 50 Gew.% bis 70 Gew.% und besonders bevorzugt von 65 Gew.%, bezogen auf das Trockengewicht der Zusammensetzung vorliegt. Derart hohe Wirkstoffkonzentrationen sind mit den aus dem Stand der Technik bekannten Verfahren nicht erreichbar, da sich hierbei die vorgenannten Probleme bei der Trocknung der aus der Zusammensetzung gebildeten Filme ergeben können. Die hochdosierten Wirkstoffe können hierbei konzentrierte Salzlösungen bilden, die sich nicht mehr trocknen lassen oder es kann sich eine ungleiche Verteilung des Wirkstoffes ergeben.

Mit dem erfindungsgemäßen Verfahren können die vorgenannten hohen Wirkstoffgehalte in Zusammensetzungen, insbesondere in Zusammensetzungen zur oralen Applikation, bevorzugt in Form eines orodispersiblen Films realisiert werden, ohne dass es zu den nachteiligen Auswirkungen, wie beispielsweise der Bildung einer konzentrierten, nicht trockenbaren Lösung kommt. In einer besonders bevorzugten Ausführungsform werden im erfindungsgemäßen Verfahren leicht wasserlösliche pharmazeutische Inhaltsstoffe formuliert, wobei bevorzugt ein organisches Lösungsmittels bzw. Lösungsmittelgemisch (B) verwendet wird, welches frei oder im wesentlichen frei von Wasser ist und wobei der wenigstens eine pharmazeutische Inhaltsstoff in diesem Lösungsmittel bzw. Lösungsmittelgemisch (B) nicht löslich oder schwer löslich ist und sich gleichmäßig in der Suspension verteilt (dispergiert). Die homogene Verteilung bleibt auch beim Trocknen der Zusammensetzung in Form eines Films erhalten. "Im wesentlichen wasserfrei" bedeutet hierbei ein Wasseranteil am Lösungsmittel bzw. Lösungsmittelgemisch (B) von kleiner als 2 % (v/v), bevorzugt kleiner als 0,5 % (v/v).

Im erfindungsgemäßen Verfahren ist des Weiteren vorgesehen, dass der Anteil des Gelbildners zwischen 5 Gew.% und 25 Gew.%, bevorzugt zwischen 10 Gew.% und 15 Gew.% und besonders bevorzugt bei 7,5 Gew.% liegt, wobei sich dieser Wert jeweils auf das Trockengewicht der Zusammensetzung bezieht. Über den Anteil an Gelbildner können auch Filmeigenschaften wie Textur, Flexibilität und Schichtdicke definiert werden.

Das erfindungsgemäße Verfahren sieht des Weiteren in einer bevorzugten Ausführungsform vor, dass ein Weichmacher Verwendung findet, dessen Anteil an der aus dem Verfahren resultierenden Zusammensetzung zwischen 2 Gew.% und 15 Gew.%, bevorzugt bei 7,5 Gew.% liegt. Der ebenfalls zusätzlich oder alternativ in dem Verfahren einsetzbare Aromastoff weist bevorzugt einen Anteil von zwischen 0 Gew.% und 10 Gew.%, bevorzugt 7 Gew.% bezogen auf das Trockengewicht der Zusammensetzung auf. Wird zusätzlich oder alternativ ein Süßungsmittel verwendet, so liegt dessen Anteil günstigerweise zwischen 0 Gew.% und 5 Gew.%, bevorzugt bei 4 Gew.% bezogen auf das Trockengewicht der Zusammensetzung. Die vorgenannten Aromastoffe bzw. Süßungsmittel dienen zur Geschmacksmaskierung bzw. dazu, die Compliance entsprechender Zusammensetzungen beispielsweise in Form orodispersibler Filme sowie aller weiteren geeigneten Darreichungsformen, beispielsweise Gele oder (Gelatine)kapseln mit Gelfüllung weiter zu erhöhen, da sie diesen einen besonders angenehmen Geschmack verleihen und/oder einen unangenehmen Geschmack maskieren.

Bei dem in dem Verfahren verwendbaren Gelbildner handelt es sich um einen Gelbildner, der in dem Lösungsmittel oder Lösungsmittelgemisch (B), d.h. bevorzugt einem Lösungsmittel oder Lösungsmittelgemisch ausgewählt aus C1- bis C5-Alkanolen, insbesondere aus Ethanol oder Isopropanol, Aceton oder Mischungen aus diesen, quellbar ist.

Als besonders bevorzugt wird der Gelbildner ausgewählt aus der Gruppe bestehend aus Hydroxypropylcellulose (HPC), Hydroxypropylmethylcellulose (HPMC) oder Methylcellulose (MC) sowie Mischungen daraus. Verwendung als Gelbildner finden ebenfalls die pharmazeutisch akzeptablen Gelbildner, die beispielsweise unter dem Handelsnamen Klucel, Klucel® E, Klucel® L, Klucel® J, Klucel® G, Klucel® M, Klucel® H bzw. deren jeweilige Mahlgrade bezeichnet mit den Bezeichnungen Klucel® EF, Klucel® LF, Klucel® JF, Klucel® GF, Klucel® MF, Klucel® HF, Klucel® EXF, Klucel® LXF, Klucel® JXF, Klucel® GXF, Klucel® MXF, Klucel® HXF, bekannt sind sowie Mischungen daraus. Die mit dem Handelsnamen Klucel® bezeichnete Hydroxypropylcellulose ist ein nicht ionischer, wasserlöslicher Celluloseether. Klucel® ist sowohl in wässrigen wie auch in organischen Lösungsmitteln löslich und weist mit herkömmlichen wasserlöslichen Cellulosepolymeren vergleichbare Thermoplastizität, Oberflächenaktivität sowie Verdickungs- und Stabilisierungseigenschaften auf. Die Erfindung bleibt nicht auf Produkte der Klucel®-Reihe beschränkt sondern umfasst gleichermaßen die Verwendung vergleichbarer Substanzen anderer Hersteller.

Je nach Einsatzgebiet der mit dem erfindungsgemäßen Verfahren herstellbaren Zusammensetzung wird der entsprechend geeignete Gelbildner ausgewählt. Über diesen können Textur, sowie die weitere Eigenschaften des Gels bezüglich Flexibilität, Trocknung und Filmbildungseignung eingestellt oder definiert werden.

Bei dem Inhaltsstoff, der in dem im erfindungsgemäßen Verfahren verwendeten Lösungsmittel bzw. Lösungsmittelgemisch (B) schwer löslich bzw. nicht löslich ist, handelt es sich bevorzugt um einen pharmazeutisch wirksamen Inhaltsstoff oder eine Mischung, umfassend wenigstens einen pharmazeutisch wirksamen Inhaltsstoff, der bzw. die wie vorstehend definiert ist.

Ein pharmazeutisch wirksamer Inhaltsstoff, der mit dem erfindungsgemäßen Verfahren formuliert werden kann, ist zum Beispiel ausgewählt aus der Gruppe bestehend aus Sumatriptan, Sildenafil, Acetylcystein, Ambroxol, Citalopram, Clopidogrel, Losartan, Mitrazapin, Valproinsäure, Verapamil und/oder pharmazeutisch akzeptable Salze davon. Des Weiteren sind Mischungen aus den vorgenannten Inhaltsstoffen und weiteren Soffen geeignet. Die genannten pharmazeutisch wirksamen Inhaltsstoffe können dabei in ihrer Reinform oder als pharmazeutisch akzeptable Salze verwendet werden. Daneben besteht auch die Möglichkeit, die Reinform eines oder mehrerer der genannten Inhaltsstoffe mit einem pharmazeutisch akzeptablen Salz eines weiteren Inhaltsstoffes im erfindungsgemäßen Verfahren zu verwenden.

In einer als besonders vorteilhaft angesehenen Ausführungsform des Verfahrens wird als Inhaltsstoff bzw. pharmazeutisch wirksamer Inhaltsstoff Sumatriptan-Succinat oder Sildenafil-Citrat verwendet. Hierbei liegt bevorzugt der Gehalt an Sumatriptan-Succinat bzw. Sildenafil-Citrat bei 30 Gew.% bis 70 Gew.%, günstigerweise bei 50 Gew.% bis 65 Gew.% Wirkstoff bezogen auf das Trockengewicht der Zusammensetzung. Besonders bevorzugt liegt die Sumatriptan-Succinat bzw. Sildenafil-Citrat umfassende Zusammensetzung dabei in Form eines orodispersiblen Films vor. Das Verfahren bietet hier besondere Vorteile, da in herkömmlichen Verfahren hohe Konzentrationen der vorgenannten Wirkstoffe nicht oder nur schwer erreicht werden können.

In den bisher bekannten Verfahren zur Bildung von entsprechenden Zusammensetzungen insbesondere in Form orodispersibler Filme, die eine Verwendung von Wasser als Lösungsmittel für einen zu formulierenden wasserlöslichen Wirkstoff vorschlagen, liegt der maximal erreichbare Anteil an Wirkstoff in der Zusammensetzung bzw. im Film, vor allem der letztgenannten Wirkstoffe Sumatriptan-Succinat bzw. Sildenafil-Citrat bei maximal 10 Gew.%. Bei höheren Konzentrationen treten die eingangs beschriebenen negativen Wirkungen insbesondere bei Trocknung von Filmen auf, sodass bisher kein zufriedenstellender Film hergestellt werden konnte. Bei der Verwendung entsprechender prozentualer Anteile an Sumatriptan-Succinat, beispielsweise im Bereich von 65 Gew.% und einer Lösung des Wirkstoffes in einem wässrigen Lösungsmittel zeigt sich, dass sich mit den bekannten Verfahren keine Filme herstellen lassen. Demgegenüber können mit dem erfindungsgemäßen wasserfreien Verfahren Zusammensetzungen bereitgestellt werden, die als orale Filme ausgebildet sein können, welche Wirkstoffanteile von 65 Gew.% und mehr, bevorzugt bis zu 80 Gew.% aufweisen können, ohne dabei die aus dem Stand der Technik bekannten Nachteile aufzuzeigen.

Der in dem erfindungsgemäßen Verfahren optional verwendete Weichmacher dient der Herabsetzung der Sprödigkeit der in Form eines Films zur Verfügung gestellten Zusammensetzung und zur Erhöhung von dessen Flexibilität. In einer bevorzugten Ausführungsform des erfindungsgemäßen Verfahrens werden die Weichmacher ausgewählt aus Glycerol und Propylenglycol. Weiterhin eignen sich als Weichmacher auch Dibutylsebacat, Triacetin, Triethylcitrat und Isopropylmyristat. Die Verwendung von Propylenglycol und Glycerol wird hierbei bevorzugt.

Das im Verfahren verwendbare Süßungsmittel ist bevorzugt ausgewählt aus der Gruppe bestehend aus Monosacchariden, Disacchariden, Polysacchariden, insbesondere Maltodextrin, Sucralose, Neotam, Alitam, Cyclamat, Sorbitol, Xylitol, Saccharin, Aspartam oder Mischungen aus vorgenannten. Über die Menge bzw. den prozentualen Anteil des Süßungsmittels kann eine Geschmacksgebung der Zusammensetzung bzw. des orodispersiblen Films durchgeführt werden. Der Geschmack kann je nach zugesetztem Süßungsmittel mehr oder weniger süß ausgeführt werden, sodass hierdurch ebenfalls, aufgrund des angenehmen süßen Geschmackes, der durch die Zugabe entsprechender Süßungsmittel erreicht wird, die Compliance der Patienten erhöht werden kann.

Neben der Zugabe von Süßungsmitteln sieht eine bevorzugte Ausführungsform des Verfahrens vor, dass ein Aromastoff in den vorgenannten prozentualen Anteilen in dem Verfahren Verwendung findet. Der Aromastoff ist dabei bevorzugt ausgewählt aus der Gruppe bestehend aus Aromastoffen mit minzig-frischem Geschmack und hierbei insbesondere aus Pfefferminzöl, Pfefferminzaroma, Menthol und/oder Levomenthol. Daneben können alternativ oder zusätzlich Aromastoffe mit sauerfrischem Geschmack, insbesondere Grapefruitaroma, Zitronen- oder Orangenaroma im erfindungsgemäßen Verfahren verwendet werden. Der Geschmack der Zusammensetzung, die beispielsweise in Form eines orodispersiblen Filmes vorliegt und das sich nach deren Einnahme einstellende Geschmackserlebnis können so für den Patienten entsprechend angenehm gestaltet werden. Neben den vorgenannten Aromastoffen bzw. Kombinationen aus diesen, kann der Aromastoff auch ausgewählt werden aus Aromastoffen mit süßlich-nussigem Geschmack, insbesondere solchen Aromastoffen, die ein Nussaroma, ein Schokoladenaroma, ein Zimt- oder Gewürzaroma oder dergleichen erzeugen. Selbstverständlich besteht auch hier die Möglichkeit einer Mischung der letztgenannten Aromastoffe mit den zuvor Beschriebenen. Neben den genannten Aromastoffen können hierbei selbstverständlich alle sonstigen, geeignet oder sinnvoll erscheinenden Aromastoffe verwendet werden, um die Geschmacksrichtung entsprechender Zusammensetzungen festzulegen oder zu definieren und hierüber dann die Compliance des Patienten zu erhöhen.

In dem erfindungsgemäßen Verfahren ist vorgesehen, dass der Inhaltsstoff (A) in einem im Verfahren verwendeten Lösungsmittel oder Lösungsmittelgemisch (B) schwer löslich oder nicht löslich ist. Als günstig wird in diesem Zusammenhang angesehen, wenn das verwendete Lösungsmittelgemisch (B) ein Gemisch von wenigstens zwei Lösungsmitteln ist. Die Lösungsmittel weisen dabei günstigerweise Anteile von 50% (v/v) bis 100% (v/v), bzw. von 0% (v/v) bis 50% (v/v), jeweils bezogen auf das Lösungsmittelgemisch (B) auf. Je nach verwendetem Lösungsmittel werden die Anteile entsprechend festgelegt.

Eine bevorzugte Ausführungsform des erfindungsgemäßen Verfahrens sieht vor, dass das Lösungsmittel oder Lösungsmittelgemisch (B) aus Ethanol und Isopropanol oder Mischungen daraus besteht. Der Anteil an Ethanol oder Isopropanol im Lösungsmittelgemisch (B) liegt dabei zwischen 0% (v/v) und 30% (v/v) bzw. zwischen 70% (v/v) und 100% (v/v). Die entsprechenden Mischungsanteile und verwendeten Lösungsmittel in dem Lösungsmittelgemisch (B), sind abhängig von dem bzw. den zu formulierenden Inhaltsstoff(en) (A). Neben den vorgenannten Alkanolen Ethanol und Isopropanol, kann das Lösungsmittel bzw. Lösungsmittelgemisch (B) auch Aceton sein, bzw. einen Acetonanteil umfassen. Eine bevorzugte Ausführungsform sieht dabei vor, dass als Lösungsmittel bzw. Lösungsmittelgemisch (B) ein Gemisch aus Aceton und Ethanol verwendet wird. Der Anteil an Aceton im Lösungsmittelgemisch (B) liegt hierbei dann insbesondere zwischen 50% (v/v) und 95% (v/v), während der Anteil an Ethanol im Lösungsmittelgemisch (B) zwischen 5% (v/v) und 50% (v/v) liegt. Selbstverständlich besteht auch die Möglichkeit, dass hier Ethanol bzw. Aceton mit einem Anteil von 100% (v/v) verwendet wird, bzw. dass eine Kombination oder eine Mischung aus Aceton und Isopropanol als Lösungsmittel bzw. Lösungsmittelgemisch (B) Verwendung findet. Hierbei liegen dann die entsprechenden Anteile an Isopropanol und Aceton in dem vorgenannten Bereich, nämlich zwischen 50% (v/v) und 95% (v/v) Aceton und einem Anteil von 5% (v/v) bis 50% (v/v) Isopropanol, jeweils bezogen auf das Lösungsmittelgemisch (B).

Das erfindungsgemäße Verfahren sieht auch einen Schritt der Bildung eines Films vor. Die Bildung des Films erfolgt dabei bevorzugt in einem Gieß-, Extrusions- oder Sprühverfahren. Die aus Schritt (ii) oder (iii) des erfindungsgemäßen Verfahrens erhältliche Suspension, umfassend wenigstens einen Inhaltsstoff (A) und wenigstens einen Gelbildner, wird einem als günstig angesehenen Filmbildungsverfahren unterzogen. Die Suspension wird dabei beispielsweise auf eine entsprechende Auflagefläche aufgegossen, auf der sich die Suspension homogen verteilt. Über die aufgegossene Menge und die Gießfläche wird dabei die Nassschichtdicke des Filmes eingestellt, bevor dieser dann einem Trocknungsverfahren, beispielsweise durch Erwärmen der Gießfläche, unterzogen wird. Gleiches gilt bei der Verwendung von Zieh- oder Extrusionsverfahren. Wird ein Sprühverfahren verwendet, so wird die Suspension auf einen entsprechenden Träger aufgesprüht. Durch die aufgesprühte Menge bzw. die Sprühdauer wird hierbei dann die Nassschichtdicke des Filmes definiert eingestellt, bevor dieser dann einem Trocknungsverfahren unterzogen wird.

Bevorzugt erfolgt die Trocknung des Films dabei durch Wärmebeaufschlagung. Die Wärmebeaufschlagung kann dabei beispielsweise durch ein Warmluftgebläse erfolgen. Es besteht selbstverständlich auch die Möglichkeit, dass der Film in einem Ofen oder einer vergleichbaren Trocknungsanlage getrocknet wird.

In dem erfindungsgemäßen Verfahren kann günstigerweise ein Film mit einer Nassschichtdicke von 400 bis 600 µm, bevorzugt von 460 bis 490 µm erzeugt werden. Der Film kann dann bevorzugt eine Trockenschichtdicke von 100 bis 200 µm, bevorzugt von 140 bis 180 µm aufweisen. Selbstverständlich kann die Dicke des Filmes auch über die Trockenschichtdicke definiert werden, wobei dann die Nassschichtdicke ohne Belang ist. Die Trockenschichtdicken, die bevorzugt vorgesehen sind, liegen zwischen 100 und 200 µm, bevorzugt zwischen 140 und 180 µm. Eine Trockenschichtdicke in den hier genannten Grenzen gewährleistet ein schnelles Zerfallen des Films im Mundraum. Hierdurch wird sichergestellt, dass der Wirkstoff vollständig vom Patienten aufgenommen wird. Selbstverständlich können auch Laminate mit geringeren oder höheren Schichtdicken vorgesehen werden, was gleichermaßen von der Erfindung umfasst ist.

Das erfindungsgemäße Verfahren bietet folgende Vorteile. Der Inhaltsstoff zeigt, aufgrund des verwendeten Lösungsmittels keine bzw. eine nur sehr geringe Löslichkeit in der Suspension. Sieht das erfindungsgemäße Verfahren einen Trocknungsschritt vor, so wird bei der Trocknung der Zusammensetzung die Ausbildung von Wirkstoffinseln bzw. nicht trockenbaren Substanzanhäufungen, die der homogenen Verteilung des Wirkstoffes in der Zusammensetzung entgegenstehen, vermieden. Bei Verwendung wasserfreier Lösungsmittel bzw. Lösungsmittelgemische und hier bevorzugt von Aceton bzw. Aceton-Ethanol-, Aceton-Isopropanol- oder Ethanol-Isopropanol-Aceton-Mischungen, lassen sich geringe Trocknungstemperaturen gegenüber wasserhaltigen Zusammensetzungen, insbesondere in Form von orodispersiblen Filmen, realisieren. Dies hat den Vorteil, dass hier geringere thermische Belastungen des oder der Inhaltsstoffe(s), insbesondere von pharmazeutisch wirksamen Inhaltsstoffen, auftreten und kein Wirkstoffverlust zu verzeichnen ist. Die geringen Trocknungstemperaturen wirken sich auch positiv auf die gegebenenfalls als Aromastoff im Verfahren verwendeten ätherischen Öle und sonstigen Aromastoffe aus, da diese durch eine geringere thermische Beaufschlagung weniger zum Zerfall bzw. zum Abbau neigen.

Aufgrund der besonders homogenen Verteilung des pharmazeutischen bzw. pharmazeutisch wirksamen Inhaltsstoffes in der durch das erfindungsgemäße Verfahren herstellbaren Zusammensetzung sind im Falle der Herstellung orodispersibler Filme hohe Beschichtungs- bzw. Filmbildungsgeschwindigkeiten möglich, da hier nicht auf Wirkstoffagglomerationen in den Suspensionen, die sich beispielsweise bei wasserbasierten Lösungsmitteln und darin gelösten Wirkstoffen ergeben, Rücksicht genommen werden muss. In dem erfindungsgemäßen Verfahren können zudem wesentlich dünnere Filme im Vergleich zu wässrigen Systemen erreicht werden, es erhöht sich dadurch die Patienten-Compliance, da entsprechend dünnere Filme leichter aufgenommen werden können und sich schneller zersetzen. Ein weiterer Vorteil des erfindungsgemäßen Verfahrens ist darin zu sehen, dass der Wirkstoff im orodispersiblen Film in ursprünglicher, nicht gelöster Form vorliegt. Hieraus ergibt sich, dass der bittere Geschmack, der bei wasserlöslichen Wirkstoffen bzw. deren Lösung und anschließenden Trocknung in wässrigen Systemen auftritt, zurückgedrängt wird. Der pharmazeutisch wirksame Wirkstoff liegt hier nicht amorph vor, sodass hier der bittere Geschmackseindruck abgemildert wird. Dies bietet den Vorteil, dass hier auf eine übermäßige Verwendung von Geschmacksmaskierungsstoffen verzichtet werden kann, was wiederum die Patienten-Compliance wesentlich erhöht.

Eine als besonders günstig angesehene Ausführungsform des erfindungsgemäßen Verfahrens umfasst die Schritte:
- Lösen wenigstens eines Aromastoffes in einem ersten Gemischbestandteil eines Lösungsmittelgemisches (B), beispielsweise in Ethanol, Isopropanol oder Aceton,
- Zugabe eines zweiten Gemischbestandteiles des Lösungsmittelgemisches (B) zu dem Gemisch aus wenigstens einem Aromastoff und dem ersten Gemischbestandteil eines Lösungsmittelgemisches (B),
- Lösen eines Süßstoffes und eines Weichmachers in dem Gemisch aus erstem Gemischbestandteil, zweitem Gemischbestandteil und Aromastoff,
- Suspendieren eines pharmazeutischen oder pharmazeutisch wirksamen Inhaltsstoffes in dem Gemisch aus erstem Gemischbestandteil des Lösungsmittelgemisches, zweitem Gemischbestandteil des Lösungsmittelgemisches, Aromastoff, Süßstoff und Weichmacher,
- Zugabe eines in dem Lösungsmittelgemisch (B) quellbaren Gelbildners zu dem Gemisch aus erstem Gemischbestandteil des Lösungsmittelgemisches (B), zweitem Gemischbestandteil des Lösungsmittelgemisches (B), Aromastoff, Süßstoff Weichmacher und pharmazeutischem oder pharmazeutisch wirksamem Inhaltsstoff,
- Quellen der Suspension,
- Bilden eines Filmes und
- Trocknen des Filmes.

Besonders bevorzugt ist das Lösungsmittel oder Lösungsmittelgemisch (B) wasserfrei oder im wesentlichen wasserfrei, wobei "im wesentlichen wasserfrei" ein Wasseranteil am Lösungsmittel bzw. Lösungsmittelgemisch (B) von kleiner als 2 % (v/v), bevorzugt kleiner als 0,5 % (v/v) bedeutet.

Die Erfindung betriff auch eine Zusammensetzung, insbesondere eine Zusammensetzung in Form eines orodispersiblen Films. Die Zusammensetzung umfasst dabei wenigstens einen wie vorstehend definierten pharmazeutischen Inhaltsstoff und wenigstens einen wie vorstehend definierten Gelbildner. Die Zusammensetzung zeichnet sich dadurch aus, dass sie bevorzugt in wässrigen Lösungsmittelsystemen, einschließlich Speichel, leicht löslich ist und daher bei der Anwendung im Mundraum schnell zerfällt.

Bei dem in der Zusammensetzung verwendeten pharmazeutischen Inhaltsstoff handelt es sich bevorzugt um wenigstens einen wie vorstehend definierten pharmazeutisch wirksamen Inhaltsstoff oder eine wie vorstehend definierte Mischung, die wenigstens einen pharmazeutisch wirksamen Inhaltsstoff umfasst. Der pharmazeutische Inhaltsstoff oder pharmazeutisch wirksame Inhaltsstoff weist dabei in dem zur Herstellung der Zusammensetzung verwendeten Lösungsmittel oder Lösungsmittelgemisch (B) bevorzugt eine Löslichkeit von weniger als 0,1 mol/l (schwer löslich bzw. nicht löslich) auf.

Die erfindungsgemäße Zusammensetzung umfasst bevorzugt einen wasserlöslichen pharmazeutisch aktiven Inhaltsstoff. Weiterhin bevorzugt ist die erfindungsgemäße Zusammensetzung frei bzw. im Wesentlichen frei von Wasser und dem zur Herstellung eingesetzten Lösungsmittel bzw. Lösungsmittelgemisch (B). Der Anteil an Lösungsmittel bzw. Lösungsmittelgemisch (B) in der fertigen Zusammensetzung beträgt weniger als 20000ppm, bevorzugt weniger als 10000ppm und besonders bevorzugt weniger als 5000ppm. Insbesondere liegt der Anteil an Lösungsmittel bzw. Lösungsmittelgemisch (B) bei 2000ppm oder weniger.

Bevorzugt ist die erfindungsgemäße Zusammensetzung eine feste Zusammensetzung und hierbei insbesondere eine feste Zusammensetzung in Form eines einschichtigen Films. Weiterhin bevorzugt zerfällt eine solche Zusammensetzung nach Kontakt mit einer wässrigen Lösung wie beispielsweise Speichel, rasch. Dabei bedeutet einschichtig, dass der Film in Form einer einzigen Schicht vorliegt, wobei die Schicht bevorzugt homogen ist. Der Film kann dabei flexibel oder nicht-flexibel sein. Vorzugsweise ist der Film, auch aufgrund der zugesetzten Weichmacher flexibel und passt sich somit gut an die Form der Mundhöhle an.

Die erfindungsgemäße Zusammensetzung in Form eines orodispersiblen Films kann als runder, beliebig gerundeter, ovaler, ellipsenförmiger, dreieckiger, viereckiger bzw. quadratischer oder rechteckiger oder vieleckiger Film vorliegen. Die Zerfallszeit der erfindungsgemäßen Zusammensetzung in der Mundhöhle beträgt 1 bis 100 Sek., bevorzugt 1 bis 50 Sek., insbesondere 1 bis 10 Sek.

### Beispiele:

### Herstellungsverfahren

### Beispiel 1:

| | |
|---|---|
| Sumatriptan-Succinat | 65g |
| Grapefruitaroma | 6,8g |
| Levomenthol | 0,2g |
| Sucralose | 4g |
| Glycerol | 4g |
| Klucel® LF | 20g |
| | 100g |
| Lösungsmittel | |
| Ethanol *) | 93,6g |
| Aceton *) | 40,1g |
| *) wird während der Herstellung fast vollständig entfernt | |

### Herstellung der Beschichtungsmasse:

Grapefruitaroma und Levomenthol werden zunächst in Aceton gelöst, anschließend wird Ethanol zugegeben und Sucralose und Propylenglycol ebenfalls in der Mischung gelöst. Als nächstes wird der Wirkstoff in die Suspension eingerührt und zuletzt der Gelbildner Klucel® LF beigefügt. Die Masse wird während mindestens 12 Stunden quellen lassen. Nach dem Quellvorgang wird eine geeignete Beschichtungsanlage mit 0,7 m/min. mit der gequollenen Suspension beschichtet und die Zusammensetzung bei einer Trocknungstemperatur von maximal 40 °C zu einem Laminat getrocknet.

Im Ausführungsbeispiel wird eine Mischung aus zwei Lösungsmitteln verwendet. Es handelt sich hierbei um die organischen Lösungsmittel Aceton und Ethanol. Der Anteil des Ethanols liegt hier bei 93,6g, während 40,1g Aceton als Lösungsmittel bzw. Gemischanteil verwendet wird. Nach dem Trocknen liegen die Restlösungsmittelgehalte in den orodispersiblen Filmen bei jeweils pharmazeutisch akzeptablen 2000 ppm für Ethanol und Aceton. Regulatorisch erlaubt sind hier Restlösungsmittelgehalte von ≥ 5000 ppm.

### Beispiel 2:

| | |
|---|---|
| Sumatriptan-Succinat | 65g |
| Grapefruitaroma | 6,8g |
| Levomenthol | 0,2g |
| Sucralose | 4g |
| Glycerol | 5g |
| Klucel® LF | 19g |
| | 100g |
| Lösungsmittel | |
| Ethanol *) | 98,8g |
| Aceton *) | 42,3g |
| *) wird während der Herstellung fast vollständig entfernt | |

### Herstellung der Beschichtungsmasse:

Grapefruitaroma und Levomenthol werden zunächst in Aceton gelöst, anschließend wird Ethanol zugegeben und Sucralose und Glycerol ebenfalls in der Mischung gelöst. Als nächstes wird der Wirkstoff in die Suspension eingerührt und zuletzt der Gelbildner Klucel® LF beigefügt. Die Masse wird während mindestens 12 Stunden quellen lassen. Nach dem Quellvorgang wird eine geeignete Beschichtungsanlage mit 0,7 m/min. mit der gequollenen Suspension beschichtet und die Zusammensetzung bei einer Trocknungstemperatur von maximal 40 °C zu einem Laminat getrocknet.

Im Ausführungsbeispiel wird eine Mischung aus zwei Lösungsmitteln verwendet. Es handelt sich hierbei um die organischen Lösungsmittel Aceton und Ethanol. Der Anteil des Ethanols liegt hier bei 98,8g, während 42,3g Aceton als Lösungsmittel bzw. Gemischanteil verwendet wird. Nach dem Trocknen liegen die Restlösungsmittelgehalte in den orodispersiblen Filmen bei jeweils 2000 ppm für Ethanol und Aceton. Regulatorisch erlaubt sind hier Restlösungsmittelgehalte von >= 5000 ppm.

### Beispiel 3 (nicht ausführbares Vergleichsbeispiel):

| | |
|---|---|
| Sumatriptan-Succinat | 65g |
| Grapefruitaroma | 6,8g |
| Levomenthol | 0,2g |
| Sucralose | 4g |
| Glycerol | 7,5g |
| HPMC (Metholose SM-100) | 9g |
| NaCl | 2g |
| Pullulan | 5g |
| Tween 80 | 0,5g |

### Herstellung der Beschichtungsmasse:

Bei diesem als Vergleichsbeispiel aufgeführten Ausführungsbeispiel wird gezeigt, dass sich vergleichbare Anteile an pharmazeutisch wirksamen Inhaltsstoff nur mit dem in der Erfindung beschriebenen Verfahren realisieren lassen. Bei dem als Vergleichsbeispiel aufgeführten Beispiel 3 wird Pullulan in der wässrigen Tween-Lösung gelöst. Levomenthol und Grapefruitaroma werden separat in einer Teilmenge Ethanol gelöst. Die Restmenge Ethanol wird der wässrigen Pullulan-Lösung zugesetzt. Dann wird die Aromalösung zugegeben. Anschließend werden der Inhaltsstoff, Glycerol und Sucralose zugesetzt. Zuletzt wird HPMC eingerührt. Die Masse wird ebenfalls, wie bereits für Beispiel 1 und 2 beschrieben, für 12 Stunden quellen gelassen. Anschließend zeigt sich, dass auch bei Trocknungstemperaturen von bis zu 80 °C ein abziehbares und weiter verarbeitbares, als Grundlage eines orodispersiblen Film dienendes Laminat nicht hergestellt werden kann.

## Patentansprüche

1. Verfahren zur Herstellung einer Zusammensetzung, insbesondere einer pharmazeutischen Zusammensetzung zur oralen Applikation, umfassend die Schritte:
(i) Bilden einer Suspension aus wenigstens einem pharmazeutischen Inhaltsstoff (A) und einem Lösungsmittel oder Lösungsmittelgemisch (B), wobei der wenigstens eine pharmazeutische Inhaltsstoff (A) in dem Lösungsmittel oder Lösungsmittelgemisch (B) eine Löslichkeit von <0,1 mol/l (schwer löslich oder nicht löslich) aufweist,
(ii) Zugabe wenigstens eines Gelbildners (C) zu der Suspension, wobei der wenigstens eine Gelbildner (C) in dem Lösungsmittel oder Lösungsmittelgemisch (B) quellbar ist, und optional
(iii) Quellen der Suspension.

2. Verfahren nach Anspruch 1, ferner umfassend die Schritte:
(iv) Bilden eines Films aus der aus Schritt (ii) oder Schritt (iii) erhältlichen Suspension und
(v) Trocknen des Films.

3. Verfahren nach einem der vorhergehenden Ansprüche, weiterhin umfassend die Zugabe wenigstens einer weiteren Substanz ausgewählt aus der Gruppe bestehend aus Aroma, Süßstoff und Weichmacher, wobei die wenigstens eine weitere Substanz in dem Lösungsmittel oder Lösungsmittelgemisch (B) leicht löslich ist.

4. Verfahren nach einem der vorhergehenden Ansprüche,
**dadurch gekennzeichnet, dass**
der pharmazeutische Inhaltsstoff (A) ein pharmazeutisch wirksamer Inhaltsstoff oder eine Mischung umfassend wenigstens einen pharmazeutisch wirksamen Inhaltsstoff ist, insbesondere wobei
- ein Anteil des wenigstens einen pharmazeutischen Inhaltsstoffes (A) zwischen 10 Gew.% und 80 Gew.%, bevorzugt zwischen 50 Gew.% und 70 Gew.%,
- ein Anteil des Gelbildners (C) zwischen 5 Gew.% und 25 Gew.%, bevorzugt bei 7,5 Gew.%,
jeweils bezogen auf das Trockengewicht der Zusammensetzung, liegt und/oder, dass
- ein Anteil des Weichmachers zwischen 2 Gew.% und 15 Gew.%, bevorzugt bei 7,5 Gew.%,
- ein Anteil des Aromastoffes zwischen 0 Gew.% und 10 Gew.%, bevorzugt bei 7 Gew.%, und/oder
- ein Anteil des Süßungsmittels zwischen 0 Gew.% und 5 Gew.%, bevorzugt bei 4 Gew.%,
jeweils bezogen auf das Trockengewicht der Zusammensetzung, liegt.

5. Verfahren nach einem der vorhergehenden Ansprüche,
**dadurch gekennzeichnet, dass**
das Lösungsmittelgemisch (B) wenigstens zwei Lösungsmittel umfasst, wobei der Anteil der Lösungsmittel im Gemisch zwischen 50% (v/v) bis 100% (v/v) beziehungsweise 0% (v/v) bis 50% (v/v) beträgt.

6. Verfahren nach einem der vorhergehenden Ansprüche,
**dadurch gekennzeichnet, dass**
der pharmazeutische Inhaltsstoff (A) in einem wässrigen Lösungsmittelsystem oder wässrigen Lösungsmittelsystemen oder Mischungen daraus eine Löslichkeit von 0,1 bis >1 mol/l (leicht löslich) aufweist und das Lösungsmittel oder Lösungsmittelgemisch (B) ausgewählt ist aus C1 -C5 Alkanolen, insbesondere Ethanol oder Isopropanol, Aceton oder Mischungen daraus und/oder, dass der pharmazeutische Inhaltsstoff (A) in einem wässrigen Lösungsmittelsystem oder wässrigen Lösungsmittelsystemen oder Mischungen daraus eine Löslichkeit von 0,1 bis >1 mol/l (leicht löslich) aufweist und das Lösungsmittel (B) ein Lösungsmittelgemisch umfassend Aceton und Ethanol, insbesondere ein Lösungsmittelgemisch umfassend Aceton und Ethanol mit einem Anteil an Aceton zwischen 50% (v/v) und 95% (v/v) und einem Anteil an Ethanol zwischen 5% (v/v) und 50% (v/v), bezogen auf das Lösungsmittelgemisch, ist.

7. Verfahren nach einem der vorhergehenden Ansprüche,
**dadurch gekennzeichnet, dass**
der Gelbildner (C) ausgewählt ist aus der Gruppe bestehend aus Hydroxypropylcellulose, Hydroxypropylmethylcellulose, Methylcellulose, Klucel®, Klucel® E, Klucel® L, Klucel® J, Klucel® G, Klucel® M, Klucel® H, Klucel® EF, Klucel® LF, Klucel® JF, Klucel® GF, Klucel® MF, Klucel® HF, Klucel® EXF,
Klucel® LXF, Klucel® JXF, Klucel® GXF, Klucel® MXF, Klucel® HXF, Ethylcellulose oder Mischungen daraus.

8. Verfahren nach einem der vorhergehenden Ansprüche,
**dadurch gekennzeichnet, dass**
der pharmazeutische Inhaltsstoff (A) ausgewählt ist aus der Gruppe bestehend aus Sumatriptan, Sildenafil, Acetylcystein, Ambroxol, Citalopram, Clopidogrel, Losartan, Mitrazapin, Valproinsäure, Verapamil, pharmazeutisch akzeptablen Salzen davon oder Mischungen daraus, und insbesondere ausgewählt ist aus Sumatriptan-Succinat und Sildenafil-Citrat, insbesondere wobei
der Gehalt an Sumatriptan-Succinat bzw. Sildenafil-Citrat bei 30-70 Gew.%, bevorzugt 50-65 Gew.%, bezogen auf das Trockengewicht der Zusammensetzung, liegt.

9. Verfahren nach einem der vorhergehenden Ansprüche 3 bis 8,
**dadurch gekennzeichnet, dass**
der Weichmacher ausgewählt ist der Gruppe bestehend aus Glycerol, Propylenglycol, Dibutylsebacat, Triacetin, Triethylcitrat und Isopropylmyristat sowie Mischungen daraus, bevorzugt aus Glycerol und Propylenglycol.

10. Verfahren nach einem der vorhergehenden Ansprüche 2 bis 9,
**dadurch gekennzeichnet, dass** die Bildung des Films in einem Gieß-, Zieh-, Extrusions- oder Sprühverfahren und die Trocknung des Films durch Wärmebeaufschlagung erfolgt und/oder
der Film eine Nassschichtdicke von 400 µm bis 600 µm, bevorzugt von 460 µm bis 490 µm und/oder eine Trockenschichtdicke von 100 µm bis 200 µm, bevorzugt von 140 µm bis 180 µm aufweist.

11. Verfahren zur Herstellung einer pharmazeutischen Zusammensetzung, insbesondere einer Zusammensetzung zur oralen Applikation, bevorzugt in Form eines orodispersiblen Films, umfassend die Schritte:
(i) Bereitstellen eines Lösungsmittels oder eines Lösungsmittelgemisches (B),
(ii) Zugabe wenigstens eines Süßstoffes, wenigstens eines Weichmachers und optional wenigstens eines Aromastoffes zu dem Lösungsmittel oder Lösungsmittelgemisch (B),
(iii) Suspendieren wenigstens eines pharmazeutischen Inhaltsstoffes (A) in dem aus Schritt (ii) erhältlichen Gemisch,
(iv) Zugabe wenigstens eines in dem Lösungsmittel oder Lösungsmittelgemisch (B) quellbaren Gelbildners (C) zu dem aus Schritt (iii) erhältlichen Gemisch,
(v) Quellen der aus Schritt (iv) erhältlichen Suspension,
(vi) Bilden eines Filmes aus der aus Schritt (v) erhältlichen Suspension,
(vii) Trocknen des Filmes,
wobei der wenigstens eine pharmazeutische Inhaltsstoff (A) in dem Lösungsmittel oder Lösungsmittelgemisch (B) eine Löslichkeit von <0,1 mol/l (schwer löslich oder nicht löslich) aufweist, und wobei das Lösungsmittel oder Lösungsmittelgemisch (B) bevorzugt
wie in einem der Ansprüche 5 bis 8 definiert ist.

12. Pharmazeutische Zusammensetzung, in Form eines orodispersiblen Films, hergestellt durch ein Verfahren gemäß einem der Ansprüche 1 bis 11, wobei der Film einen Restgehalt des Lösungsmittels bzw. der Lösungsmittel (B) von weniger als 5000ppm, bevorzugt weniger als 2000ppm bezogen auf das Trockengewicht des Films aufweist und der wenigstens eine pharmazeutisch aktive Inhaltsstoff ausgewählt ist aus der Gruppe bestehend aus Sumatriptan, Sildenafil, Acetylcystein, Ambroxol, Citalopram, Clopidogrel, Losartan, Mitrazapin, Valproinsäure, Verapamil, pharmazeutisch akzeptablen Salzen davon öder Mischungen daraus, insbesondere ausgewählt ist aus der Gruppe bestehend aus Sumatriptan-Succinat und Sildenafil-Citrat, und wobei der Anteil des wenigstens einen pharmazeutisch aktiven Inhaltsstoffes zwischen 50 Gew.% und 70 Gew.% liegt und der wenigstens eine pharmazeutische Inhaltsstoff (A) in Wasser oder in einem wässrigen Lösungsmittelgemisch, einschließlich Speichel eine Löslichkeit von 0,1 bis >1 mol/l (leicht löslich) aufweist und in dem für die Herstellung des Films verwendeten Lösungsmittel oder Lösungsmittelgemisch (B), insbesondere ausgewählt aus C1 -C5 Alkanolen, bevorzugt Ethanol oder Isopropanol, Aceton oder Mischungen daraus, eine Löslichkeit von <0,1 mol/l (schwer löslich oder nicht löslich) aufweist ist.

13. Pharmazeutische Zusammensetzung nach Anspruch 12, **gekennzeichnet durch** einen Wasseranteil der pharmazeutischen Zusammensetzung von weniger als 0,5 Gew.% und einen Anteil an Lösungsmittel ausgewählt aus C1 -C5 Alkanolen, insbesondere Ethanol oder Isopropanol, Aceton oder Mischungen daraus von 2000ppm oder weniger.

## Claims

1. A method for producing a composition, in particular a pharmaceutical composition for oral administration, comprising the steps:
(i) Forming a suspension of at least one pharmaceutical ingredient (A) and a solvent or solvent mixture (B), wherein the at least one pharmaceutical ingredient (A) has a solubility of <0.1 mol/L (sparingly soluble or insoluble) in the solvent or solvent mixture (B),
(ii) Addition of at least one gelling agent (C) to the suspension, wherein the at least one gelling agent (C) is swellable in the solvent or solvent mixture (B), and optionally
(iii) Swelling of the suspension.

2. The method according to claim 1, additionally comprising the steps:
(iv) Forming a film of the suspension obtainable from step (ii) or step (iii), and
(v) Drying the film.

3. The method according to any one of the preceding claims, additionally comprising the addition of at least one additional substance selected from the group consisting of flavoring, sweetener and softening agent, wherein the at least one additional substance is readily soluble in the solvent or solvent mixture (B).

4. The method according to any one of the preceding claims,
**characterized in that**
the pharmaceutical ingredient (A) is a pharmaceutical active ingredient or a mixture comprising at least one pharmaceutically active ingredient, in particular wherein
it contains:
the at least one pharmaceutical ingredient (A) in an amount between 10% by weight and 80% by weight, preferably between 50% by weight and 70% by weight,
the gelling agent (C) in an amount between 5% by weight and 25% by weight, preferably 7.5% by weight,
each based on the dry weight of the composition and/or
the softening agent in an amount between 2% by weight and 15% by weight, preferably 7.5% by weight,
the flavoring substance in an amount between 0% by weight and 10% by weight, preferably 7% by weight and/or
the sweetener in an amount between 0% by weight and 5% by weight, preferably 4% by weight
each based on the dry weight of the composition.

5. The method according to any one of the preceding claims,
**characterized in that**
the solvent mixture (B) comprises at least two solvents, wherein the solvents are present in the mixture in an amount between 50% (v/v) and 100% (v/v) and/or 0% (v/v) and 50% (v/v).

6. The method according to any one of the preceding claims,
**characterized in that**
the pharmaceutical ingredient (A) has a solubility of 0.1 to >1 mol/L (readily soluble) in an aqueous solvent system or aqueous solvent systems or mixtures thereof, and the solvent or solvent mixture (B) is selected from C₁-C₅ alkanols, in particular ethanol or isopropanol, acetone or mixtures thereof, and/or the pharmaceutical ingredient (A) has a solubility of 0.1 to >1 mol/L (readily soluble) in an aqueous solvent system or aqueous solvent systems or mixtures thereof, and the solvent (B) is a solvent mixture comprising acetone and ethanol, in particular a solvent mixture comprising acetone and ethanol with an amount of acetone between 50% (v/v) and 95% (v/v) and an amount of ethanol between 5% (v/v) and 50% (v/v), based on the solvent mixture.

7. The method according to any one of the preceding claims,
**characterized in that**
the gelling agent (C) is selected from the group consisting of hydroxypropyl cellulose, hydroxypropylmethyl cellulose, methyl cellulose, Klucel^{®}, Klucel^{®} E, Klucel^{®} L, Klucel^{®} J, Klucel^{®} G, Klucel^{®} M, Klucel^{®} H, Klucel^{®} EF, Klucel^{®} LF, Klucel^{®} JF, Klucel^{®} GF, Klucel^{®} MF, Klucel^{®} HF, Klucel^{®} EXF, Klucel^{®} LXF, Klucel^{®} JXF, Klucel^{®} GXF, Klucel^{®} MXF, Klucel^{®} HXF, ethyl cellulose or mixtures thereof.

8. The method according to any one of the preceding claims,
**characterized in that**
the pharmaceutical ingredient (A) is selected from the group consisting of sumatriptan, sildenafil, acetylcysteine, ambroxol, citalopram, clopidogrel, losartan, mirtazapine, valproic acid, verapamil, pharmaceutically acceptable salts thereof or mixtures thereof, and in particular is selected from sumatriptan succinate and sildenafil citrate, in particular wherein the sumatriptan succinate content and/or sildenafil citrate content is 30-70% by weight, preferably 50-65% by weight, based on the dry weight of the composition.

9. The method according to one of the preceding claims 3 to 8, **characterized in that**
the softening agent is selected from the group consisting of glycerol, propylene glycol, dibutyl sebacate, triacetin, triethyl citrate and isopropyl myristate as well as mixtures thereof, preferably glycerol and propylene glycol.

10. The method according to one of the preceding claims 2 to 9, **characterized in that**
formation of the film takes place in a casting, drawing, extrusion or spray process and the drawing of the film takes place by heat treatment and/or the film has a wet layer thickness of 400 µm to 600 µm, preferably from 460 µm to 490 µm and/or has a dry layer thickness of 100 µm to 200 µm, preferably 140 µm to 180 µm.

11. The method for producing a pharmaceutical composition, in particular a composition for oral administration, preferably in the form of an orodispersible film, comprising the steps:
(i) Supplying a solvent or solvent mixture (B),
(ii) Adding at least one sweetener, at least one softening agent and optionally at least one flavoring substance to the solvent or solvent mixture (B),
(iii) Suspending at least one pharmaceutical ingredient (A) in the mixture obtainable from step (ii),
(iv) Adding to the mixture obtainable from step (iii) at least one gelling agent (C) that is capable of swelling in the solvent or solvent mixture (B),
(v) Swelling the suspension obtainable from step (iv),
(vi) Forming a film of the suspension obtainable from step (v),
(vii) Drying the film,
wherein the at least one pharmaceutical ingredient (A) has a solubility of <0.1 mol/L (sparingly soluble or insoluble) in the solvent or solvent mixture (B) and wherein the solvent or solvent mixture (B) is preferably defined as in one of claims 5 to 8.

12. A pharmaceutical composition in the form of an orodispersible film produced by a method according to one of claims 1 to 11, wherein the film has a residual solvent content and/or solvent mixture content (B) of less than 5000 ppm, preferably less than 2000 ppm, based on the dry weight of the film, and the at least one pharmaceutically active ingredient is selected from the group consisting of sumatriptan, sildenafil, acetylcysteine, ambroxol, citalopram, clopidogrel, losartan, mirtazapine, valproic acid, verapamil, pharmaceutically acceptable salts thereof or mixtures thereof, in particular being selected from the group consisting of sumatriptan succinate and sildenafil citrate, and wherein the amount of the at least one pharmaceutically active ingredient is between 50% by weight and 70% by weight, and the at least one pharmaceutical ingredient (A) has a solubility of 0.1 to >1 mol/L (readily soluble) in water or in an aqueous solvent mixture including saliva, and has a solubility of <0.1 mol/L (sparingly soluble or insoluble) in the solvent or solvent mixture (B) used to produce the film, in particular selected from C₁-C₅ alkanols, preferably ethanol or isopropanol, acetone or mixtures thereof.

13. The pharmaceutical composition according to claim 12, **characterized by** a water content of the pharmaceutical composition of less than 0.5% by weight and an amount of solvent selected from C₁-C₅ alkanols, in particular ethanol or isopropanol, acetone or mixtures thereof, of 2000 ppm or less.

## Revendications

1. Procédé de fabrication d'une composition, en particulier d'une composition pharmaceutique pour une administration orale, comprenant les étapes suivantes :
(i) formation d'une suspension à partir d'au moins un ingrédient pharmaceutique (A) et d'un solvant ou mélange de solvants (B), le au moins un ingrédient pharmaceutique (A) présentant dans le solvant ou mélange de solvants (B) une solubilité de <0,1 mol/l (difficilement soluble ou insoluble),
(ii) addition d'au moins un gélifiant (C) à la suspension, le au moins un gélifiant (C) étant apte à gonfler dans le solvant ou mélange de solvants (B), et facultativement
(iii) gonflement de la suspension.

2. Procédé selon la revendication 1, comprenant en outre les étapes suivantes :
(iv) formation d'un film à partir de la suspension provenant de l'étape (ii) ou de l'étape (iii) et
(v) séchage du film.

3. Procédé selon l'une des revendications précédentes, comprenant en outre l'addition d'au moins une autre substance choisie dans le groupe consistant en arôme, édulcorant et plastifiant, la au moins une autre substance étant légèrement soluble dans le solvant ou mélange de solvants (B).

4. Procédé selon l'une des revendications précédentes,
**caractérisé par le fait que**
l'ingrédient pharmaceutique (A) est un ingrédient pharmaceutiquement actif ou un mélange comprenant au moins un ingrédient pharmaceutiquement actif, en particulier dans lequel:
- une proportion du au moins un ingrédient pharmaceutique (A) est comprise entre 10 % en poids et 80 % en poids, de préférence entre 50 % en poids et 70 % en poids,
- une proportion du gélifiant (C) est comprise entre 5 % en poids et 25 % en poids, et de préférence égale à environ 7,5 % en poids,
chacune sur la base du poids sec de la composition et/ou **par le fait que** :
- une proportion du plastifiant est comprise entre 2 % en poids et 15 % en poids, et de préférence égale à 7,5 % en poids,
- une proportion de l'arôme est comprise entre 0 % en poids et 10 % en poids, et de préférence égale à 7 % en poids, et/ou
- une proportion de l'édulcorant est comprise entre 0 % en poids et 5 % en poids, et de préférence égale à 4 % en poids,
chacune sur la base du poids sec de la composition.

5. Procédé selon l'une des revendications précédentes,
**caractérisée en ce que**
le mélange de solvants (B) comprend au moins deux solvants, la proportion des solvants dans le mélange étant comprise respectivement entre 50 % (p/p) et 100 % (p/p) et entre 0 % (p/p) et 50 % (p/p).

6. Procédé selon l'une des revendications précédentes,
**caractérisé par le fait que**
l'ingrédient pharmaceutique (A) présente, dans un système de solvants aqueux ou des systèmes de solvants aqueux ou des mélanges de ceux-ci, une solubilité de 0,1 à >1 mol/l (légèrement soluble) et le solvant ou mélange de solvants (B) est choisi parmi les alcanols en C₁-C₅, en particulier l'éthanol ou l'isopropanol, l'acétone ou leurs mélanges et/ou en ce que l'ingrédient pharmaceutique (A) présente, dans un système de solvant aqueux ou des systèmes de solvants aqueux ou des mélanges de ceux-ci, une solubilité de 0,1 à >1 mol/l (légèrement soluble) et le solvant (B) est un mélange de solvants comprenant de l'acétone et de l'éthanol, en particulier un mélange de solvants comprenant de l'acétone et de l'éthanol avec une proportion de l'acétone entre 50 % (p/p) et 95 % (p/p) et une proportion de l'éthanol entre 5 % (p/p) et 50 % (p/p), sur la base du mélange de solvants.

7. Procédé selon l'une des revendications précédentes,
**caractérisé par le fait que**
le gélifiant (C) est choisi dans le groupe consistant en:
hydroxypropylcellulose, hydroxypropylméthylcellulose, méthylcellulose, Klucel®, Klucel®E, Klucel®L, Klucel®J, Klucel®G, Klucel®M, Klucel®H, Klucel®EF, Klucel®LF, Klucel®JF, Klucel®GF, Klucel®MF, Klucel®HF, Klucel®EXF, Klucel®LXF, Klucel®JXF, Klucel®GXF, Klucel®MXF, Klucel®HXF, éthylcellulose ou leurs mélanges.

8. Procédé selon l'une des revendications précédentes,
**caractérisée en ce que**
l'ingrédient pharmaceutique (A) est choisi dans le groupe consistant en sumatriptan, sildénafil, acétylcystéine, ambroxol, citalopram, clopidogrel, losartan, mitrazapine, acide valproïque, vérapamil, leurs sels pharmaceutiquement acceptables ou leurs mélanges, et est en particulier choisi parmi le succinate de sumatriptan et le citrate de sildénafil,
la teneur en succinate de sumatriptan ou en citrate de sildénafil étant de 30-70 % en poids, de préférence de 50-65 % en poids, sur la base du poids sec de la composition.

9. Procédé selon l'une des revendications 3 à 8,
**caractérisée en ce que**
le plastifiant est choisi dans le groupe consistant en glycérol, propylène glycol, sébaçate de dibutyle, triacétine, citrate de triéthyle et myristate d'isopropyle, ainsi que leurs mélanges, de préférence parmi le glycérol et le propylène glycol.

10. Procédé selon l'une des revendications 2 à 9,
**caractérisé par le fait que** la formation du film s'effectue dans un procédé par coulée, par étirage, par extrusion ou par pulvérisation et le séchage du film s'effectue par application de chaleur et/ou
le film présente une épaisseur de couche humide de 400 µm à 600 µm, de préférence de 460 µm à 490 µm et/ou une épaisseur de couche sèche de 100 µm à 200 µm, de préférence de 240 µm à 180 µm.

11. Procédé de fabrication d'une composition pharmaceutique, en particulier d'une composition pour une administration orale, de préférence sous la forme d'un film orodispersible, comprenant les étapes suivantes :
(i) préparation d'un solvant ou d'un mélange de solvants (B),
(ii) addition d'au moins un édulcorant, d'au moins un plastifiant et facultativement d'au moins un arôme au solvant ou mélange de solvants (B),
(iii) mise en suspension d'au moins un ingrédient pharmaceutique (A) dans le mélange provenant de l'étape (ii),
(iv) addition d'au moins un gélifiant (C) apte à gonfler dans le solvant ou mélange de solvants (B) au mélange provenant de l'étape (iii),
(v) gonflement de la suspension provenant de l'étape (iv),
(vi) formation d'un film à partir de la suspension provenant de l'étape (v),
(vii) séchage du film,
le au moins un ingrédient pharmaceutique (A) présentant dans le solvant ou mélange de solvants (B) une solubilité de <0,1 mol/l (difficilement soluble ou insoluble) et le solvant ou mélange de solvants (B) étant, de préférence, tel que défini dans l'une des revendications 5 à 8.

12. Composition pharmaceutique sous la forme d'un film orodispersible, fabriquée par un procédé selon l'une des revendications 1 à 11, dans lequel le film présente une teneur résiduelle en solvant ou mélange de solvants (B) inférieure à 5000 ppm, de préférence inférieure à 2000 ppm, sur la base du poids sec du film et le au moins un ingrédient pharmaceutiquement actif étant choisi dans le groupe consistant en : sumatriptan, sildénafil, acétylcystéine, ambroxol, citalopram, clopidogrel, losartan, mitrazapine, acide valproïque, vérapamil, leurs sels pharmaceutiquement acceptables ou leurs mélanges, en particulier choisi dans le groupe consistant en le succinate de sumatriptan et le citrate de sildénafil, et dans lequel la proportion du au moins un ingrédient pharmaceutiquement actif est comprise entre 50 % en poids et 70 % en poids et le au moins un ingrédient pharmaceutique (A) présente, dans l'eau ou un mélange de solvants aqueux, y compris la salive, une solubilité de 0,1 à >1 mol/l (légèrement soluble) et, dans le solvant ou mélange de solvants (B) utilisé pour la fabrication du film, en particulier choisi parmi les alcanols en C₁-C₅, de préférence l'éthanol ou l'isopropanol, l'acétone ou leurs mélanges, une solubilité de <0,1 mol/l (difficilement soluble ou insoluble).

13. Composition pharmaceutique selon la revendication 13, **caractérisée par** une teneur en eau de la composition pharmaceutique inférieure à 0,5 % en poids et par une proportion de solvant choisi parmi les alcanols en C₁-C₅, en particulier l'éthanol ou l'isopropanol, l'acétone ou leurs mélanges, de 2000 ppm ou moins.
